# EUROPEAN PATENT APPLICATION

(11) **EP 3 626 175 A1**
(43) Date of publication of application: **25.03.2020**
(21) Application number: 18195439.7
(22) Date of filing: 19.09.2018
(51) Int. Cl.: A61B 6/03, A61B 6/00, G21K 1/10, A61B 6/02

(54) **BEAM SHAPING FILTER FOR CBCT**

(71) Applicant: Koninklijke Philips N.V., 5656 AG Eindhoven (NL)
(72) Inventor: SCHAEFER, Dirk, 5656 AE Eindhoven (NL); VAN DE HAAR, Peter George, 5656 AE Eindhoven (NL); HUMMEL, Erik, 5656 AE Eindhoven (NL); WITHAGEN, Petrus Johannes, 5656 AE Eindhoven (NL)
(74) Representative: de Haan, Poul Erik

(57) **Abstract**

In a first aspect, the present invention relates to a beam shaping filter (1) for use in a cone beam computed tomography system. The filter comprises a radiation attenuating element for positioning between an x-ray source of the cone beam computed tomography system and an object to be imaged. The radiation attenuation as function of position in at least a part (2) of the radiation attenuating element is rotationally symmetric with respect to a point of rotational symmetry (3).

## Description

### FIELD OF THE INVENTION

The invention relates to the field of computed tomography, and, more specifically, to x-ray beam shaping and flux equalization techniques in computed tomography. More specifically it relates to a beam shaping filter, a cone beam computed tomography system and a method.

### BACKGROUND OF THE INVENTION

In computed tomography (CT), a collimated x-ray source is typically used to project an X-ray beam through an object to be imaged, such as a patient. The x-ray beam, attenuated by the object, is then received by an x-ray detector array. The source and detector are typically rotated together around the object to obtain images from multiple angles to enable a tomographic reconstruction, e.g. of cross-sectional images through the object.

Beam shaping devices, such as bowtie filters, can be used in CT imaging to decrease the dynamic range of x-ray beam intensities when attenuated by an imaged object. By limiting the dynamic range over the x-ray detector, detector saturation and artefacts associated therewith can be avoided or reduced, x-ray scattering can be reduced and the spatial noise distribution can be conditioned to be more homogenous. Furthermore, the x-ray radiation dose that a patient receives can be reduced by such beam shaping devices, e.g. without compromising image quality. The shape of the filter is typically configured to compensate for the variation in thickness of the imaged object, e.g. of the patient's body. Thus, the shape is chosen such that the x-ray beam intensity profile approximately matches the attenuation profile of the object. The beam shaping device may typically comprise a compensator, such as a bowtie filter, for positioning in between the x-ray source and the object to be imaged.

Bowtie designs are used in clinical applications for CT scanners, e.g. with circular and/or helical scanning trajectories. A bowtie filter has a low attenuation in the center and increasing attenuation towards higher fan-angles in trans-axial direction. The bowtie filter, as known in the art, may comprise a bowtie-shaped element made of metal, e.g. a machined piece of aluminium, or other suitable material, such as a polymer.

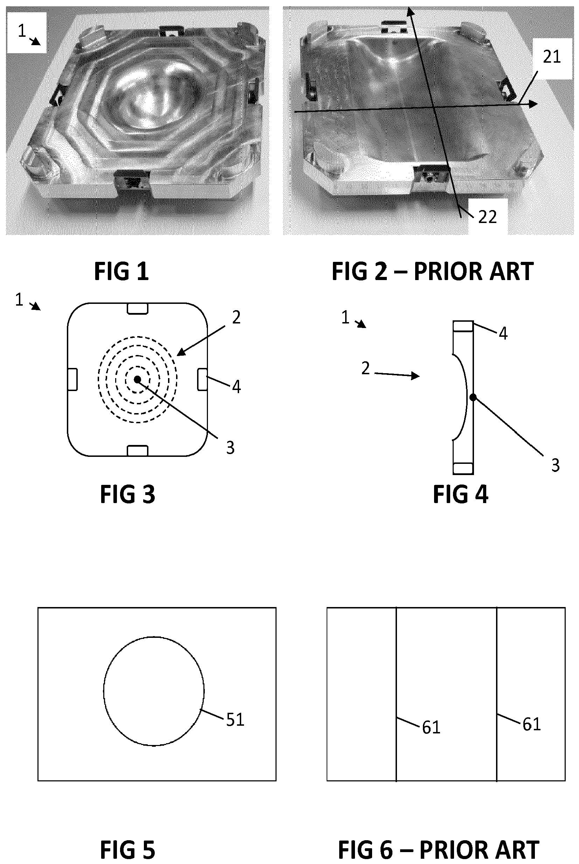

It is known in the art to use a one-dimensional (1D) bowtie filter shape, as illustrated in FIG 2. Such ID bowtie filter has an attenuation profile, e.g. a thickness profile, that is non-constant, i.e. bow-shaped, along a single direction 21 and substantially constant in the direction 22 perpendicular to that direction, hence the reference to 1D. The single direction of varying attenuation, e.g. varying thickness, may be typically oriented in a direction that is orthogonal to the longitudinal axis of the patient, e.g. such that the filter presents a substantially constant profile along the longitudinal axis. The shape of this 1D profile may be adapted to different body regions, to different patient sizes and/or to optimize the dose distribution in the patient.

Furthermore, bowtie filters having a non-constant profile in the direction that is intended for, in operation, being oriented along the longitudinal axis of the patient are also known in the art. For example, dynamic bowtie filters are known in the art that consist of a controllable array of beam shaper elements. For example, to adjust the attenuation profile during or between scans, the attenuation in each element can be controlled individually, for example by adjusting gas pressures. However, it is a disadvantage of such dynamic filters that the pixelation of the array can be coarse and/or that the filter assembly may require an impractical gain calibration.

It is also known to move a static filter to change the effective attenuation profile relative to the beam shape. For example, in WO 2015/022599, an adjustable filter assembly is disclosed that comprises a first filter element shaped as a background-wedge for attenuating x-rays having a large aperture and a second filter element, constructed to create a ridge, that can be rotated (or adjusted) with respect to the first filter element to adapt to different helical pitch values.

However, the use of bowties in cone-beam CT (CBCT) systems, such as C-arm systems and integrated imaging systems in radiotherapy, typically requires a rotational gain calibration to renormalize the incoming flux due to the bowtie shape and wobble.

### SUMMARY OF THE INVENTION

It is an object of embodiments of the present invention to provide good and efficient means and methods for beam shaping in cone-beam CT imaging.

It is an advantage of embodiments of the present invention that means and/or methods in accordance with embodiments of the present invention may be particularly suitable for beam shaping to reduce a dose to a patient, to reduce a dynamic range to prevent or reduce x-ray detector saturation, to reduce or avoid imaging artefacts and/or to obtain a more homogeneous spatial noise distribution.

It is an advantage of embodiments of the present invention that means and/or methods in accordance with embodiments of the present invention may be particularly suitable for beam shaping in imaging of the head of a patient, e.g. in brain imaging, such as imaging of neurovasculature.

It is an advantage of embodiments of the present invention that means and/or methods in accordance with embodiments of the present invention may be particularly suitable for beam shaping in a cone-beam CT imaging system adapted for acquiring a sequence of projections for tomographic reconstruction by rotating around at least two axes, e.g. in a C-arm cone beam system that is configured to acquire images by rotating around multiple non-parallel axes of rotation, such as by following an acquisition trajectory that comprises simultaneous propeller and roll movements.

It is an advantage of embodiments of the present invention that a good approximation of the inverse attenuation profile of the human head can be achieved, e.g. particularly when considering an acquisition trajectory that comprises rotations around at least two non-collinear axes, e.g. simultaneous roll, pitch and/or yaw motions.

It is an advantage of embodiments of the present invention that a robust gain calibration can be achieved of a cone-beam CT system comprising a filter in accordance with embodiments.

It is an advantage of embodiments of the present invention that few or no bowtie artefacts can be achieved, e.g. in a dual-axis C-arm CBCT system, even when using relative in-plane rotations between the bowtie filter and the detector.

It is an advantage of embodiments of the present invention that good beam shaping can be achieved without requiring a dynamically adjustable beam shaper, e.g. without requiring a translatable or rotatable (e.g. relative to the beam axis) filter or filter component and/or without requiring an array of controllable filter elements.

The above objective is accomplished by a method and device according to the present invention.

In a first aspect, the present invention relates to a beam shaping filter for use in a cone beam computed tomography system. The filter comprises a radiation attenuating element for positioning between an x-ray source of the cone beam computed tomography system and an object to be imaged. The radiation attenuation as function of position in at least a part of the radiation attenuating element is rotationally symmetric with respect to a point of rotational symmetry.

In a beam shaping filter in accordance with embodiments of the present invention, the radiation attenuation may be a non-constant function of the radial distance to the point of rotational symmetry.

In a beam shaping filter in accordance with embodiments of the present invention, the radiation attenuation may be a monotonously increasing function of the radial distance to the point of rotational symmetry.

In a beam shaping filter in accordance with embodiments of the present invention, the point of rotational symmetry may be the center of the radiation attenuating element.

In a beam shaping filter in accordance with embodiments of the present invention, at least said part of the radiation attenuating element may have a locally varying thickness to provide the radiation attenuation as function of position.

In a beam shaping filter in accordance with embodiments of the present invention, the radiation attenuating element may be composed of aluminum, molybdenum and/or teflon.

A beam shaping filter in accordance with embodiments of the present invention may comprise a fastener or mechanical connector for mechanically connecting the beam shaping filter to the cone beam computed tomography system such that the radiation attenuating element is positioned between the x-ray source and the object to be imaged, in operation of the system, and such that the point of rotational symmetry coincides with a central beam axis of a beam of ionizing radiation emitted by the x-ray source in operation of the cone beam computed tomography system.

In a second aspect, the present invention relates to a cone beam computed tomography system comprising the beam shaping filter in accordance with embodiments of the first aspect of the present invention.

A cone beam computed tomography system in accordance with embodiments of the present invention may comprise an x-ray source and an x-ray detector configured to jointly rotate around an examination volume.

In a cone beam computed tomography system in accordance with embodiments of the present invention, the x-ray source and the x-ray detector may be configured to jointly rotate around the examination volume over a first angular range with respect to a first axis of rotation and over a second angular range with respect to a second axis of rotation that is not collinear with the first axis of rotation.

In a cone beam computed tomography system in accordance with embodiments of the present invention, the x-ray source and the x-ray detector may be mounted on a C-arm.

A cone beam computed tomography system in accordance with embodiments of the present invention may be adapted for acquiring image data (e.g. projection images) while following a substantially isocentric dual-axis trajectory.

In a cone beam computed tomography system in accordance with embodiments of the present invention, the beam shaping filter may be configured to remain stationary with respect to the x-ray source in operation of the system.

In a third aspect, the present invention relates to a method for imaging at least part of a subject's head. The method comprises positioning a radiation attenuating element of a beam shaping filter between the subject's head and an x-ray source emitting a cone beam of x-ray beams. The radiation attenuation of the radiation attenuating element as function of position (e.g. a position on a major surface of the radiation attenuating element) is rotationally symmetric with respect to a point of rotational symmetry. The method comprises detecting a plurality of projection images of the cone beam attenuated by the radiation attenuating element and the subject's head using an x-ray detector. The method comprises moving the x-ray source and the x-ray detector while detecting the plurality of projection images by following a substantially isocentric dual-axis trajectory.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### BRIEF DESCRIPTION OF THE DRAWINGS

FIG 1 shows a photograph of an ionizing radiation beam shaping filter in accordance with embodiments of the present invention.
FIG 2 shows a photograph of a prior-art non-dynamic ID bowtie filter.
FIG 3 schematically shows a frontal view of an exemplary beam shaping filter in accordance with embodiments of the present invention.
FIG 4 schematically shows a cut view of an exemplary beam shaping filter in accordance with embodiments of the present invention.
FIG 5 schematically shows an exemplary iso-attenuation line corresponding to the attenuation as function of position of a beam shaping filter in accordance with embodiments of the present invention.
FIG 6 schematically shows an exemplary iso-attenuation line corresponding to the attenuation as function of position of a prior-art non-dynamic 1D bowtie filter.
FIG 7 shows an image in which the iso-attenuation curve (e.g. as illustrated in FIG 5) of a filter in accordance with embodiments of the present invention is overlaid on a projection image of a human head in a standard orientation.
FIG 8 shows an image in which the iso-attenuation curve (e.g. as illustrated in FIG 6) of a prior-art ID filter is overlaid on the projection image that was also used in FIG 7.
FIG 9 shows an image in which the iso-attenuation curve (e.g. as illustrated in FIG 5) of a filter in accordance with embodiments of the present invention is overlaid on a projection image of a human head in a rotated orientation.
FIG 10 shows an image in which the iso-attenuation curve (e.g. as illustrated in FIG 6) of a prior-art ID filter is overlaid on the projection image of a human head in the rotated orientation of FIG 9.
FIG 11 schematically shows exemplary iso-attenuation curves corresponding to the attenuation as function of position of a beam shaping filter in accordance with embodiments of the present invention for a plurality of projections along an iso-centric dual-axis trajectory with approximately +/- 30° relative in-plane angle.
FIG 12 schematically shows exemplary iso-attenuation curves corresponding to the attenuation as function of position of a prior-art non-dynamic 1D bowtie filter for the plurality of projections along the iso-centric dual-axis trajectory of FIG 11.
FIG 13 schematically illustrates a system in accordance with embodiments of the present invention.
FIG 14 illustrates a method in accordance with embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### DETAILED DESCRIPTION OF ILLUSTRATIVE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a beam shaping filter for use in a cone beam computed tomography system. The beam shaping filter comprises a radiation attenuating element for positioning between an x-ray source of the cone beam computed tomography system and an object to be imaged. The radiation attenuating element comprises at least a part in which the radiation attenuation as function of position is rotationally symmetric with respect to a point of rotational symmetry.

The radiation attenuating element (e.g. or the entire beam shaping filter) may be composed of a metal or a polymer or some other suitable material. Exemplary materials include Aluminum (Al), Molybdenum (Mo) and Teflon.

Referring to FIG 3 and FIG 4, an exemplary beam shaping filter 1 in accordance with embodiments of the present invention is shown, respectively in a frontal view and a cut view. Furthermore, FIG 1 shows a photograph of a beam shaping filter 1 in accordance with embodiments of the present invention.

The filter 1 is adapted for use in a cone beam computed tomography system. The beam shaping filter comprises (or consists of) a radiation attenuating element for positioning between an x-ray source of the cone beam computed tomography system and an object to be imaged.

The filter 1 may comprise a fastener and/or mechanical connector and/or support 4, as known in the art, e.g. in prior-art bowtie filters, for mechanically connecting the filter to the cone beam computed tomography system, e.g. by engaging a filter holder of the system, such that the radiation attenuating element is thereby positioned between the x-ray source and the object to be imaged (e.g. between the source and a detector of system, e.g. particularly substantially closer to the source than to the detector).

The filter 1 may be adapted for being used as a static beam-shaping filter, e.g. a non-dynamic beam-shaping filter. For example, the fastener and/or mechanical connector and/or support 4 may be adapted for connecting to a part, e.g. a filter holder, of the CBCT system that is fixed with respect to the x-ray source. For example, the fastener and/or mechanical connector and/or support 4 may be unsuitable for receiving a control signal or force to dynamically reconfigure the radiation attenuation of the filter as function of position.

In a further embodiment, the filter 1 may be integrated into a filter-wheel (as known in the art) in the collimation unit, which allows the automatic changing between bowties and filters depending on pre-selected imaging protocols.

The frontal view of FIG 3 may correspond to a view as presented when a viewing axis to obtain the view is parallel to the primary beam axis of an ionizing radiation beam emitted by the x-ray source when the radiation attenuating element is, in use, positioned as intended between the x-ray source and the object to be imaged. Likewise, the side view of FIG 4 may correspond to a view as presented when a viewing axis to obtain the view is perpendicular to this primary beam axis.

The radiation attenuating element may be substantially planar, e.g. may have a dominant shape of a plane, even though this shape may locally deviate from this dominant planar shape, e.g. to accommodate variations in thickness to provide a radiation attenuation as function of position for shaping an ionizing radiation beam when emitted by the x-ray source.

The radiation attenuating element comprises at least a part 2 in which the radiation attenuation as function of position is rotationally symmetric with respect to a point of rotational symmetry 3, e.g. is radially symmetric.

The radiation attenuation as function of radial distance from the point of rotational symmetry may be non-constant, such as an increasing function, e.g. a monotonously increasing function.

The part 2 may have a locally varying thickness, e.g. as illustrated in FIG 4, to provide the radiation attenuation as function of position. For example, the thickness as function of position over a major (e.g. front or back) surface may be rotationally symmetric, e.g. radially symmetric, with respect to the point of rotational symmetry 3. However, while variations in thickness may offer an advantageously simple approach to locally varying the attenuation, embodiments of the present invention are not limited thereto. For example, local variations in the attenuation properties may be equally achieved by variations in material properties, such as density, atomic number and such.

The part 2 may be a central part of the radiation attenuating element. The point of rotational symmetry 3 may be the center, or near the center, of the radiation attenuating element.

The radiation attenuating element may be adapted for positioning between an x-ray source of the cone beam computed tomography system and an object to be imaged such that the point of rotational symmetry 3 substantially coincides with a central beam axis of a beam of ionizing radiation emitted by the x-ray source in operation of the system.

As will be discussed further in detail hereinbelow, the rotational symmetry may be particularly advantageous for imaging of the head of a patient, e.g. cranial imaging and/or neuro-imaging. For example, a good approximation of the inverse attenuation profile of the human head can be achieved. This rotational symmetry is even more advantageous for imaging of the head in a CBCT system adapted for acquiring a sequence of projections by rotating around at least two axes, for example a C-arm CBCT system that is configured to acquire images by rotating around multiple non-parallel axes of rotation, such as by following an acquisition trajectory that comprises simultaneous roll and pitch, simultaneous roll and yaw, simultaneous pitch and yaw or simultaneous roll, pitch and yaw movements. For example, the CBCT system may be adapted for following an acquisition trajectory that comprises rotations around at least two non-collinear axes, e.g. simultaneous roll, pitch and/or yaw motions.

Typically, the radiation attenuation as function of position, e.g. an attenuation profile or attenuation map, is measured to form of a gain map, as known in the art, such that acquired projection data, e.g. the measured patient imaging data, can be normalized. A schematic gain map illustration of an iso-attenuation curve 51, e.g. at 40% of the maximum intensity, corresponding to a filter 1 in accordance with embodiments of the present invention, is shown in FIG 5. Such circular iso-attenuation curves are indicative of the radial symmetry of the attenuation as function of position of the attenuating element of the filter. For the sake of comparison, FIG 6 shows a schematic illustration of the iso-attenuation curve 61 (e.g. 40% of max intensity) of a prior-art ID bow-tie filter, e.g. corresponding to the prior-art filter shown in FIG 2.

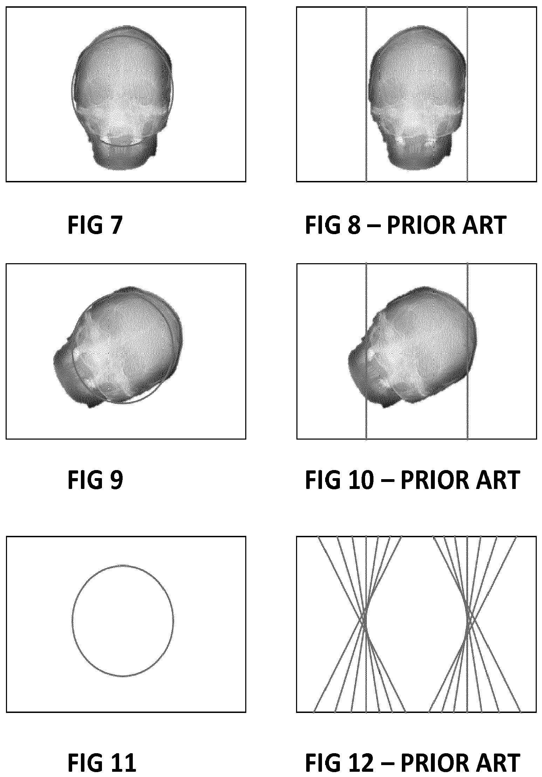

As already mentioned hereinabove, the radially symmetric shape of the attenuation map is particularly suited to approximate the attenuation of a human head, e.g. better suited for this purpose than a conventional prior-art ID bow-tie filter as shown in FIG 8, in a typical acquisition orientation, e.g. an orientation as would typically be used during a conventional acquisition sequence in which a circular trajectory around the head is followed (e.g. around the dorsoventral axis). Particularly, referring to FIG 7, the iso-attenuation curve (or at least one of all the possible iso-attenuation curves) of the filter in accordance with embodiments of the present invention conforms better to the shape of the head, e.g. to the iso-intensity curve(s) of the acquired projection image, than the prior-art ID bowtie filter.

Furthermore, when the filter in accordance with embodiments of the present invention is used in a CBCT system that is adapted for following an acquisition trajectory comprising rotations around at least two non-collinear axes, e.g. a 'dual-axis' (of rotation) trajectory, the advantages of embodiments of the present invention in imaging the head are even more pronounced. Such dual-axis (or multiple-axis) trajectories can advantageously improve the image quality in CBCT, because sufficient data in the Tuy-sense can be acquired in this manner, in contrast to the more conventional circular arc acquisitions, i.e. using single-axis (of rotation) trajectories.

For example, such dual-axis (or multiple-axis) acquisitions may be characterized by projection images in which the head is rotated in the sagittal plane and/or in the coronal plane, while a conventional 'single-axis' acquisitions would consist, typically, of only projections images for various projection angles around the head corresponding to rotations in the transverse (or axial) plane. This is illustrated in FIG 9, which shows that the radially symmetric shape of the attenuation map can approximate the attenuation of a human head better than a conventional prior-art 1D bow-tie filter, as shown in FIG 10, for a rotated acquisition orientation that could be used during a dual-axis acquisition sequence.

A poor match of the head anatomy in dual-axis trajectories using a prior-art 1D bowtie filter might be mitigated by a relative in-plane rotation of the 1D bowtie profile w.r.t. the patient axis, while keeping the detector fixed with respect to the C-arm. For example, the one-dimensional axis of mirror symmetry of the bowtie filter could be aligned to match the orientation of the inferior-superior axis of the head. However, this requires a more complex approach in which the bow-tie filter (and/or the CT system) has to be adapted to allow a rotation of the bow-tie filter. Furthermore, the required alignment would require an additional alignment step to be performed.

Nonetheless, a filter in accordance with embodiments of the present invention would still have an advantage when an iso-centric dual-axis acquisition trajectory and a relative in-plane rotation are combined. In standard helical or circular CT or CBCT, a single gain map suffices to re-normalize the acquired patient projections, because the bowtie shadow (viewed in the projections) is identical for all trajectory positions. Therefore, the iso-attenuation lines for all projections are the same, e.g. as illustrated in FIG 6. Therefore, rotational gain images can be averaged over large angular ranges to reduce Poisson noise in the gain projection and account for small system drifts or wobble along the trajectory.

However, the use of iso-centric dual-axis trajectories and a relative in-plane rotation of the bowtie filter and the detector would lead to varying gain projections for every source position along the trajectory when using a conventional bow-tie filter as known in the art, e.g. as illustrated by FIG 12, whereas the use of a radial symmetric filter results in gain projections that are independent of the acquisition angle, see FIG 11. Slight deviations from the iso-centricity can therefore be determined in the conventional approach, e.g. using a rotational gain calibration with large angular averaging/binning. The prior-art ID bowtie filter would, on the other hand, require a separate gain map for each projection angle.

In a second aspect, the present invention relates to a cone beam computed tomography system comprising a beam shaping filter in accordance with embodiments of the first aspect of the present invention.

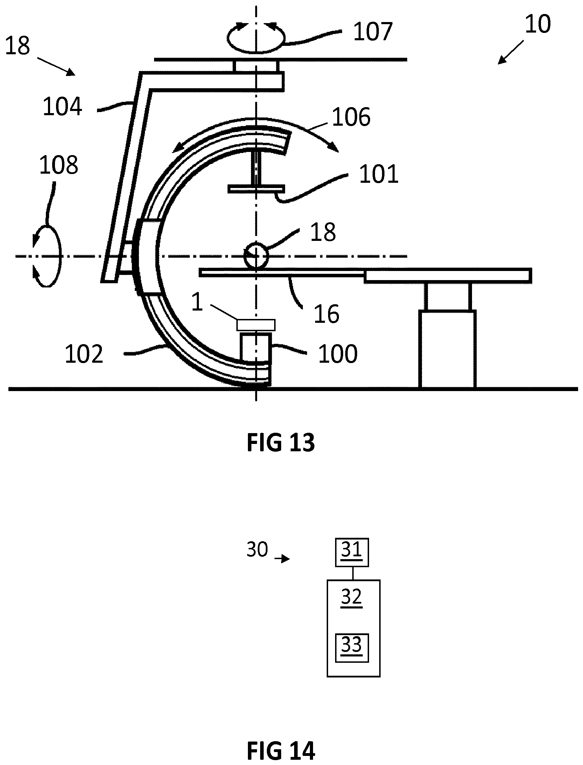

Referring to FIG 13, a cone beam computed tomography system 10 in accordance with embodiments of the present invention is shown. The CBCT system 10 may comprise an X-ray source 100 and an x-ray detector 101. The x-ray source 100 and the x-ray detector 101 may be configured such as to enable a joint rotation of the source and detector around an examination volume 18, e.g. an examination volume in which a subject to be imaged is positioned in use of the system. For example, the detector and the source may be mounted on a rotatable gantry.

The x-ray source 100 may be adapted for emitting a cone-beam of x-rays across the examination volume 18. The x-ray detector 101 may be adapted for receiving the cone-beam when transmitted from the source through the examination volume. For example, the x-ray detector may be a flat panel x-ray detector.

In a preferred embodiment of the present invention, the x-ray source 100 and the x-ray detector 101 may be configured such as to enable a joint rotation of the source and detector around the examination volume over a first angular range with respect to a first axis of rotation and over a second angular range with respect to a second axis of rotation (i.e. which is not collinear with the first axis of rotation). For example, joint rotations of the source and detector around at least two, e.g. three, mutually non-collinear axes may be provided, e.g. a primary rotation 106, a secondary rotation 108 and a tertiary rotation 107.

For example, the cone beam computed tomography system may be adapted for acquiring imaging data while following a substantially isocentric dual-axis trajectory.

The cone-beam computed tomography system 10 may comprise (or consist of) a C-arm imaging system adapted for CBCT scanning. For example, the x-ray source 100 and the x-ray detector 101 may be mounted on a C-arm 102, which may provide a first degree of freedom of rotation (primary rotation 106). The C-arm may be rotatably mounted on an L-arm 104 to provide a second degree of freedom of rotation (secondary rotation 108). The L-arm 104 may be rotatably mounted to (or supported by) a fixed anchor point, e.g. a floor, wall or ceiling, to provide a third degree of freedom of rotation (tertiary rotation 107).

The cone-beam computed tomography system 10 may comprise (or consist of) an on-board imager of a radiation therapy device.

The system may comprise a subject support 16 for supporting a subject, e.g. a patient, to be imaged. For example, a rotatable gantry, e.g. a rotatable part of the C-arm 102, may be rotatable around the subject support 16.

The system may comprise other elements as known in the art, such as a data processing and/or control unit. For example, the system 10 may comprise a CT acquisition module for receiving detected x-ray data from the detector 101. The system may comprise a tomographic reconstruction module for reconstructing a tomographic representation of the imaged subject based on the detected x-ray data.

The beam shaping filter 1 may be positioned between the x-ray source 100 and an object to be imaged, e.g. between the x-ray source 100 and the examination volume 18, or may be configured for being positioned as such in operation of the system. For example, the system 10 may comprise a filter holder onto which the beam shaping filter may be removably attached to position the beam shaping filter 1 between the source and the object to be imaged, or the system may comprise an actuator for controllably bringing the beam shaping filter into such position and/or for controllable removing the beam shaping filter from such position when it is not needed.

The beam shaping filter 1 may remain stationary with respect to the x-ray source 100 in operation of the system, e.g. the beam shaping filter may not be, or may not be configured as, a dynamic beam shaping filter.

In a third aspect, the present invention relates to a method for imaging at least part of a subject's head. Referring to FIG 14, an exemplary method 30 in accordance with embodiments of the present invention is shown. The method 30 comprises positioning 31 a radiation attenuating element of a beam shaping filter between the subject's head and an x-ray source emitting a cone beam of x-ray beams. The radiation attenuation of the radiation attenuating element as function of position is rotationally symmetric with respect to a point of rotational symmetry, e.g. which may coincide with a central beam axis of the cone beam. The method comprises detecting 32 a plurality of projection images of the cone beam attenuated by the radiation attenuating element and the subject's head by using an x-ray detector. The method comprises moving 33 the x-ray source and the x-ray detector while detecting the plurality of projection images by following a substantially isocentric dual-axis trajectory.

## Claims

1. A beam shaping filter (1) for use in a cone beam computed tomography system, the filter comprising a radiation attenuating element for positioning between an x-ray source of the cone beam computed tomography system and an object to be imaged, in which the radiation attenuation as function of position in at least a part (2) of the radiation attenuating element is rotationally symmetric with respect to a point of rotational symmetry (3).

2. The beam shaping filter of claim 1, wherein said radiation attenuation in said part (2) is a non-constant function of the radial distance to the point of rotational symmetry (3).

3. The beam shaping filter of claim 2, wherein said radiation attenuation in said part (2) is a monotonously increasing function of the radial distance to the point of rotational symmetry (3).

4. he beam shaping filter of any of the previous claims, wherein said point of rotational symmetry (3) is the center of said radiation attenuating element.

5. The beam shaping filter of any of the previous claims, wherein said part (2) has a locally varying thickness to provide said radiation attenuation as function of position.

6. The beam shaping filter of any of the previous claims, wherein said radiation attenuating element is composed of aluminum, molybdenum and/or teflon.

7. The beam shaping filter of any of the previous claims, comprising a fastener or mechanical connector (4) for mechanically connecting the beam shaping filter to said cone beam computed tomography system such that the radiation attenuating element is thereby positioned between the x-ray source and the object to be imaged and said point of rotational symmetry (3) coincides with a central beam axis of a beam of ionizing radiation emitted by the x-ray source in operation of said cone beam computed tomography system.

8. A cone beam computed tomography system (10) comprising the beam shaping filter (1) of any of the previous claims.

9. The cone beam computed tomography system (10) of claim 8, comprising an x-ray source (100) and an x-ray detector (101) configured to jointly rotate around an examination volume (18).

10. The cone beam computed tomography system (10) of claim 9, wherein said x-ray source (100) and said x-ray detector (101) are configured to jointly rotate around the examination volume over a first angular range with respect to a first axis of rotation and over a second angular range with respect to a second axis of rotation that is not collinear with the first axis of rotation.

11. The cone beam computed tomography system (10) of claim 10, wherein the x-ray source (100) and the x-ray detector (101) are mounted on a C-arm (102).

12. The cone beam computed tomography system (10) of claim 11 or claim 12, wherein said system (10) is adapted for acquiring imaging data while following a substantially isocentric dual-axis trajectory.

13. The cone beam computed tomography system (10) of any of the claims 9 to 12, wherein said beam shaping filter (1) remains substantially stationary with respect to said x-ray source (100) in operation of the system.

14. A method for imaging at least part of a subject's head, the method comprising:
positioning a radiation attenuating element of a beam shaping filter between the subject's head and an x-ray source emitting a cone beam of x-ray beams, wherein the radiation attenuation of said radiation attenuating element as function of position is rotationally symmetric with respect to a point of rotational symmetry,
detecting a plurality of projection images of said cone beam attenuated by said radiation attenuating element and said subject's head using an x-ray detector, and
moving said x-ray source and said x-ray detector while detecting said plurality of projection images by following a substantially isocentric dual-axis trajectory.
